(19) Europäisches Patentamt
European Patent Office
Office européen des brevets

(11) **EP 4 335 361 A1**

(12) **EUROPEAN PATENT APPLICATION**

(43) Date of publication:
**13.03.2024 Bulletin 2024/11**

(21) Application number: **22194618.9**

(22) Date of filing: **08.09.2022**

(51) International Patent Classification (IPC):
**A61B 5/026** (2006.01)  **A61B 5/00** (2006.01)

(52) Cooperative Patent Classification (CPC):
**A61B 5/0261; A61B 5/7257; A61B 5/7267**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **WISSEL, Tobias**
  **Eindhoven (NL)**
• **VERKRUIJSSE, willem**
  **Eindhoven (NL)**

(74) Representative: **Philips Intellectual Property & Standards**
**High Tech Campus 52**
**5656 AG Eindhoven (NL)**

(54) **PROCESSING LASER SPECKLE IMAGES**

(57)    A mechanism for processing one or more laser speckle images. A spatial frequency transform is used to transform the one or more laser speckle images into spatial frequency data. The spatial frequency data is used to define a plurality of blood velocity indicators, which each indicate an amount of blood moving at a particular velocity within an illuminated region of interest.

FIG. 2

**Description**

TECHNICAL FIELD

The present disclosure relates generally to the field of laser speckle images, and in particular to the processing and analysis of laser speckle images.

BACKGROUND

An amount of microvascular dysfunction in subjects having peripheral arterial disease (PAD) is increasingly recognized as an important factor influencing their clinical outcome. Microvascular dysfunction affects the capability of the vasculature to react to changing demands for oxygen in the parenchyma. Pathophysiological root causes of vascular dysfunction are often complex to establish, and are thought to result from interplay of different conditions including capillary ischemia, structural micro-angiopathy or compromised hyperaemic responses.

One useful indicator of microvascular dysfunction is blood velocity. Laser speckle contrast imaging (LSCI), sometimes simply labelled laser speckle imaging (LSI), has historically been used to construct a perfusion index K that changes more or less linearly with the average blood velocity.

The measurement principle is based on observing a speckle pattern of light that is reflected from a (light) perfused surface of a subject, which is represented by a laser speckle image. The speckle pattern in the laser speckle image originates from interfering light backscattered from a distribution of scattering particles. This not only includes a tissue matrix, but also any red blood cells residing in the tissue. The movement of the latter leads to a decorrelation of the reflected electric field, where the decorrelation time constant is proportional to the speed of the red blood cells. Thus, the speckle pattern in a laser speckle image will change responsive to the speed of the red blood cells, i.e., the blood velocity.

This results in an exposure time dependent spatial blurring of the speckle pattern in the laser speckle image. This means that it is possible to compute a perfusion index K(I), for a laser speckle image of a region of interest, using the standard deviation of the pixel values $\sigma(I)$, normalized by the average intensity $<I>$ of the pixel values across the area of interest, as follows:

$$K(I) = \frac{\sigma(I)}{<I>} \qquad (1)$$

There is an ongoing desire to provide additional and/or more accurate clinically information. In particular, it would be advantageous to improve knowledge about the blood velocity in regions of interest of a subject, to aid in the diagnosis, assessment and treatment of microvascular dysfunction and/or PAD.

SUMMARY OF THE INVENTION

The invention is defined by the claims.

There is proposed a computer-implemented method for processing one or more laser speckle images, each laser speckle image being an image containing a speckle pattern produced by the reflection of light illuminating a region of interest of a subject, the computer-implemented method comprising: processing the one or more laser speckle images using a spatial frequency transform, to thereby produce spatial frequency data; and defining, using the spatial frequency data, a plurality of blood velocity indicators, each blood velocity indicator indicating or representing an amount of blood moving at a respective, different blood velocity within the region of interest.

The present disclosure recognizes that it would be particularly advantageous to determine indicators that represent an amount or intensity of blood that is moving at different velocities. In particular, this can be used to determine or represent a blood velocity distribution. It is recognized that a blood velocity distribution is particularly indicative of a hyperaemic response of a subject, so that changes in a blood velocity distribution can indicate a changed or compromised hyperaemic responses.

Thus, it would be advantageous to produce data that changes responsive to changes in blood velocity distribution. This would allow changes in hyperaemic responses to be identified. Such data could also be processed to determine one or more characteristics of the subject, as there is a causal relationship between such data and the blood velocity distribution.

Is it also recognized that there will be an expected velocity distribution arising from blood travelling in sized vessels, i.e., vessels of different calibers or internal diameters. Variations from an expected velocity distribution could reflect pathological patterns or concerns, e.g., the presence of shunting or static microvascular dysfunction (e.g. resulting from micro-angiopathy).

Approaches are based on the realization that spatial frequency information of a (single) laser speckle image contains information of the blood velocity distribution. The invention proposes not to compute a single quantity K(I) from a laser speckle image or spatial speckle pattern, but to compute the spectrum across all spatial frequencies and use this information to directly define blood velocity indicators representing an amount or magnitude of blood moving at different blood velocities, thereby establishing more detailed perfusion state information.

Considered spectrally, the spatial standard deviation that has historically been used to calculate the perfusion index K(I) is the average across the power spectrum. By contrast, the present proposal is to not average the power spectrum of spatial frequencies (as implicitly done when computing K(I)), but rather evaluate the magnitudes of different spatial frequencies. This provides additional and more useful clinical information, e.g., for pinpointing or identifying relevant blood velocity information.

It will be appreciated that the blood velocity indicators will together provide information on a velocity distribution within the region of interest. A blood velocity distribution provides insights on where the blood flows, as velocity is smaller with decreased vessel caliber size. This information is useful for assessing or understanding the condition of a subject/patient. For instance, if a disease is causing increased shunting from the capillary bed, the proportion of slower moving red blood cells will be lower as blood is bypassing in larger caliber AV anastomoses. This can be identified in changes to the blood velocity indicators. Similarly, if dynamic adjustment of blood flow by vasomotor mechanisms (vessel contraction / dilation) or sphincter regulation (shunts) is impaired, then this is observable not only on the behavior of the average velocity, but also in the velocity distribution.

In some examples, the step of processing the one or more laser speckle images to produce spatial frequency data comprises converting the one or more laser speckle images into a spatial power spectrum identifying, for each of a plurality of spatial frequencies, a power of the spatial frequency in the one or more laser speckle images. Each power in the spatial power spectrum can act as a respective blood velocity indicator.

In particular, the magnitudes of the spatial frequencies (i.e., the powers of the spatial power spectrum) can together act as a multivariate marker or vector for the blood velocity distribution and perfusion of the tissue

In some examples, the step of processing the laser speckle image to produce spatial frequency data comprises: converting each laser speckle image into an initial spatial power spectrum identifying, for each of a plurality of spatial frequencies, a power of the spatial frequency in the laser speckle image; defining a plurality of spatial frequency ranges; and producing the spatial power spectrum by, for each of the plurality of spatial frequency ranges, combining the powers of the spatial frequencies, of each initial spatial power spectrum, that fall in the spatial frequency range to produce a power for the spatial power spectrum. The combining may, for instance, comprise summing, adding or averaging the powers of the spatial frequencies (in the same spatial frequency range).

This approach effectively transfers the initial spatial power spectrum into the spatial power spectrum by combining spatial frequency intervals into a fixed number of bins. This approach allows for higher signal-to-noise ratios, whilst also compensating for different sampling resolutions in the spatial frequency domain by standardizing it into a fixed grid or fixed set of spatial frequency ranges.

Thus, the defined plurality of spatial frequency ranges may be predetermined.

The computer-implemented method may further comprise processing one or more of the blood velocity indicators using a computer-implemented method to determine one or more values for one or more characteristics of the subject, wherein the one or more characteristics comprise at least one of: a blood velocity distribution and/or a clinical parameter such as a pathology prognosis.

This approach recognizes that the magnitudes of the spatial frequencies can also be converted to an improved or other measure (e.g., a more clinically interpretable measure), using one or more statistical models trained to carry out this conversion, to provide data of better interpretability by a clinician or further processing system. This is because the spatial frequencies are responsive or change responsive to changes in blood velocities, blood velocity distribution and resultant clinical parameters (such as pathology prognosis or subject well-being).

The computer-implemented model may comprise a machine-learning model.

The computer-implemented method may comprise: obtaining a time-varying signal representing a temporal variation of blood flow in the region of interest; transforming the time-varying signal to the frequency domain; defining a set of frequency magnitudes which contains magnitudes of different frequency components or frequency ranges of the time-varying signal; and using the blood velocity indicators and the defined set of frequency magnitudes to determine one or more parameters of the illuminated region of interest.

Example parameters include the blood velocity distribution and/or the value of the clinical parameter(s) previously described.

The computer-implemented method may further comprise a laser speckle image defining process comprising, for each laser speckle image: obtaining an initial laser speckle image, being an image containing a speckle pattern produced by the reflection of light illuminating a region of interest of a subject, wherein the initial laser speckle image contains the laser speckle image and has a higher resolution than the laser speckle image; processing the initial laser speckle image to identify one or more parts of the initial laser speckle image that represent the region of interest; and extracting the identified one or more parts of the initial laser speckle image to define the laser speckle image.

In some examples, the step of processing the initial laser speckle image comprises: identifying, as heterogeneous parts, any parts of the initial laser speckle image that represent heterogeneous regions of the subject that contain one or more blood capillaries and one or more of: an artery and a vein; and excluding the identified heterogeneous parts of the initial laser speckle image from the identified one or more parts of the initial laser speckle image that represent the region of interest.

If included in a laser speckle image, heterogeneous regions can lead to misinterpretation as the spectral analysis of a small patch will always assume a periodic continuation of the observed pattern. Therefore, for improved accuracy, severe heterogeneity needs to be detected and either rejected or masked out.

If the laser speckle image includes spatial heterogeneity (e.g. the imaged region contains a larger vessel surrounded by tissue), then the spatial frequency transform would assume that signal jumps due to the vessel would re-appear regularly outside the field of view and could include frequencies in the spectrum, which are more related to the position of the vessel in the current image rather than the speckle pattern.

In some examples, the step of processing the initial laser speckle image comprises identifying one or more parts of the initial laser speckle image that contain one or more desired textual features of the initial laser speckle image as the parts of the initial laser speckle image that represent the region of interest.

The one or more laser speckle images may comprise a plurality of laser speckle images having different exposure times. Images recorded with different exposure times will contain blood velocity information with different dynamic ranges. This improves the robustness and accuracy of blood velocity indicators generated using multiple laser speckle images. In particular, with different exposure times it possible to determine or otherwise account for the decorrelation time of backscattered electromagnetic waves.

There is also proposed a computer program product comprising code which, when executed by a processor circuit, causes the processor circuit to perform the steps of any herein described method. There is also proposed a non-transitory computer-readable medium or data carrier comprising or carrying the computer program product.

There is also proposed a processing system processing one or more laser speckle images, each laser speckle image being an image containing a speckle pattern produced by the reflection of light illuminating a region of interest of a subject.

The processing system is configured to: process the one or more laser speckle images using a spatial frequency transform, to thereby produce spatial frequency data; and define, using the spatial frequency data, a plurality of blood velocity indicators, each blood velocity indicator indicating or representing an amount of blood moving at a respective, different blood velocity within the region of interest.

The skilled person would be readily capable of adapting the processing system to perform any herein described method.

There is also proposed a laser speckle imaging system comprising: the processing system; and a laser speckle device configured to capture the one or more laser speckle images.

Any advantages of the computer-implemented method according to the present invention analogously and similarly apply to the system and computer program herein disclosed.

It will be appreciated by those skilled in the art that two or more of the above-mentioned options, implementations, and/or aspects of the invention may be combined in any way deemed useful.

BRIEF DESCRIPTION OF THE DRAWINGS

For a better understanding of the invention, and to show more clearly how it may be carried into effect, reference will now be made, by way of example only, to the accompanying drawings, in which:

Figure 1 illustrates a laser speckle imaging system comprising a processing system according to an embodiment;
Figure 2 illustrates a method for processing one or more laser speckle images;
Figure 3 illustrates a method for generating processed data using one or more laser speckle images;
Figure 4 illustrates the effect of changes in the effect of increase flow on a spatial power spectrum of the one or more laser speckle images;
Figure 5 illustrates a method for generating output data using one or more laser speckle images and a time-varying signal;
Figure 6 illustrates a method for obtaining and processing one or more laser speckle images;
Figure 7 illustrates approaches for obtaining one or more laser speckle images; and
Figure 8 illustrates a processing system according to an embodiment.

DETAILED DESCRIPTION OF THE EMBODIMENTS

The invention will be described with reference to the figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the figures are merely schematic and are not drawn to scale. It should also be understood that the same reference numerals are used throughout the figures to indicate the same or similar parts.

The invention provides a mechanism for processing one or more laser speckle images. A spatial frequency transform is used to transform the one or more laser speckle images into spatial frequency data. The spatial frequency data is used to define a plurality of blood velocity indicators, which each indicate an amount of blood moving at a particular velocity within an illuminated region of interest.

In the context of the present disclosure, the "subject" may be alternatively labelled the "current subject" or "subject under investigation". This contrasts with a "historic subject". When used alone, the term "frequency" generally refers to a time-derived frequency, in contrast to a "spatial frequency".

Embodiments may be used in a number of clinical scenarios in which information of blood velocity in regions of interest of a subject would be useful. Example scenarios include the diagnosis, assessment and treatment of microvascular dysfunction and/or PAD (including initial and follow-up appointments).

Another use-case scenario for proposed approaches is their use during perfusion challenges such as occlusion triggered or thermally stimulated hyperaemia experiments. Here, changes in the blood velocity indicators (e.g., the spectral distribution of the speckle function) act as an indicator for changes in the blood velocity distribution. The latter probes the capabilities of the vasculature to respond and adapt to external stimuli and changing demands.

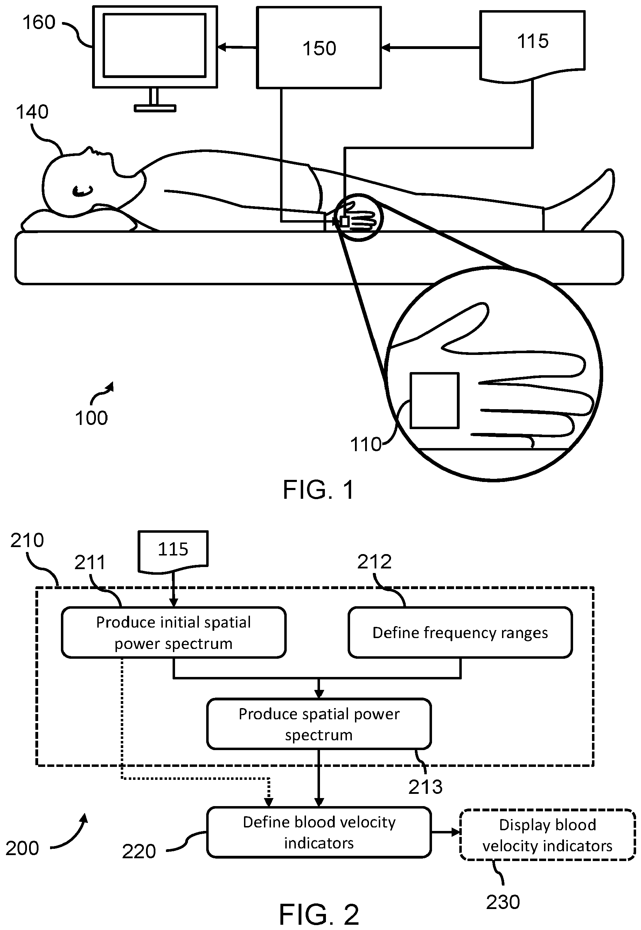

Figure 1 conceptually illustrates a laser speckle imaging system 100 according to an embodiment. The laser speckling imaging system 100 comprises a laser speckle device 110 and a processing system 150, which is itself an embodiment of the invention.

The laser speckle device 110 is configured to produce one or more laser speckle images 115 of a subject 140, e.g., a plurality of laser speckle images 115 of the subject 140. A laser speckle image 115 is an image containing a speckle pattern produced by the reflection of light illuminating a region of interest of a subject. Approaches for generating laser speckle images of a subject, using a laser speckle device, are well-established in the art.

As an example, the laser speckle device 110 may be configured to irradiate coherent light, e.g., red or near-infrared light, onto the tissue and observe the backscattered speckle pattern (as the laser speckle image 115). This can be realized using a light emitting system and camera system (or other optical sensor) or attachable electrodes. The selection of a camera system or attachable electrodes may be dependent upon on the clinical scenario.

For instance, the laser speckle device 110 may comprise and use an optical sensor or camera system during diagnosis or follow-up for PAD patients (due to its low cost implementation). Such an optical sensor or camera system may comprise a CCD camera and/or CMOS sensors. As another example, a contact-based system, e.g. with attachable electrodes, may be used in an intra-procedural setting to confirm treatment impact.

Each laser speckle image will be associated with a particular exposure time T. An exposure time T indicates a length of time over which the laser speckle image is captured, i.e., the length of time for which reflected light is collected, as is well known in the art of image acquisition.

The processing system 150 is configured to process the laser speckle image(s) 115 to produce output data.

In particular, the processing system 150 is configured to process the laser speckle image(s) using a spatial frequency transform, to thereby produce spatial frequency data. The processing system then uses the spatial frequency data to define a plurality of blood velocity indicators.

The plurality of blood velocity indicators, extracted from the spatial frequency data, will carry information about the distribution of blood velocities within the imaged region. This provides useful information for a clinician that improves their understanding of the region of interest and the subject.

The information carried by the plurality of blood velocity indicators may be considered to be in an encoded format, e.g., not provide direct information about, or a 1:1 correspondence with, the velocity distribution. Nonetheless, the plurality of blood velocity indicators will still change responsive to changes in the velocity distribution, allowing inferences to be made from the produced data and/or changes identified.

The processing system 150 may be configured to perform one or more pre-processing steps on the laser speckle image(s) before using the spatial frequency transform. Similarly, the processing system 150 may be configured to perform one or more post-processing steps on the plurality of blood velocity indicators, e.g., to map or convert the blood velocity indicators into some target quantity or prediction (e.g., actual velocity distribution, a perfusion state or outcome measure).

More detailed examples and explanations of possible pre- and post-processing steps or procedures are provided later in this disclosure.

In some examples, the processing system 150 may be configured to control the operation of the laser speckle device, e.g., to control how and/or when laser speckle images are captured by the laser speckle device. For instance, the processing system 150 may be able to control one or more capture properties of the laser speckle device, e.g., exposure time, the time at which the laser speckle image is captured, the illumination intensity, the illumination location and so on.

More detailed examples and explanations of such procedures are provided later in this disclosure.

The processing system 150 produces output data, which may include the plurality of blood velocity indicators themselves and/or processed data. The processed data is data produced by performing one or more post-processing steps on (at least) the blood velocity indicators.

The laser speckle imaging system 100 may comprise a user interface 160, such as a display or screen. The processing system 150 may be configured to control the user interface 160 to provide a visual representation of the output data (e.g., the plurality of blood velocity indicators and/or the processed data). Approaches for controlling a user interface 160 to provide a visual representation of data are well established in the art, and are not explained for the sake of conciseness.

Figure 2 is a flowchart illustrated a computer-implemented method 200 according to an embodiment. The computer-implemented method may be carried out or performed by the processing system 150, illustrated in Figure 1.

The computer-implemented method comprises a process 210 of processing the one or more laser speckle images using a spatial frequency transform, to thereby produce spatial frequency data. The process 210 here performs steps that convert the one or more laser speckle images into a spatial power spectrum. The spatial power spectrum identifies, for each of the plurality of spatial frequencies, a power of the spatial frequency in the laser speckle image(s).

Spatial frequency transforms are well known in the art. A spatial power spectrum produced by a spatial frequency transform effectively identifies or defines how pixel values of an image vary in space. Effectively, a spatial frequency transform is a multi-dimensional Fourier transform that decomposes a variation of pixel value or intensity over distance (e.g., compared to a traditional frequency transform that decomposes a variation of pixel value over time).

One example of a spatial frequency transform is a fast Fourier transform (FFT) or a short-time fast Fourier transform (STFFT). However, other suitable spatial frequency transforms could be used, e.g., any suitable Fourier-based transform.

In some approaches, performing a spatial frequency transform comprises performing a multidimensional spatial frequency transform. This produces a multidimensional spatial power spectrum. Process 210 may then convert the multidimensional spatial spectrum into a ID (one-dimensional) spatial power spectrum by treating the data along each of a plurality of angles within the multidimensional spatial spectrum as an initial ID spatial spectrum. It can be assumed that the measured spectral features should not (significantly) differ on the orientation of the multidimensional spatial power spectrum. The initial ID spatial spectrums can be combined (e.g., by summing or averaging) to produce a combined ID spatial spectrum that acts as the output of the spatial frequency transform, i.e., as the spatial power spectrum.

The procedure for generating a 1D spatial power spectrum from a multidimensional image can be labelled a multi-to-single dimension spatial frequency transform. Other procedures for generating a 1D spatial power spectrum from a multidimensional image will be apparent to the skilled person.

One technique for producing a spatial power spectrum is to simply perform a spatial frequency transform on each laser speckle image to produce a respective spatial power spectrum.

If there is more than one laser speckle image (thereby producing multiple spatial power spectrums) then the produced spatial power spectrums can be combined (e.g., summed or averaged) to produce the spatial power spectrum to be output by process 210. This approach is particularly advantageous if the laser speckle images are obtained at a same point in a cardiac cycle (and/or other rhythmic cycle that affects the imaged region, such as a respiratory cycle). Alternatively, if there is more than one laser speckle image, then the produced spatial power spectrums can be concatenated together. This approach is particularly advantageous if the laser speckle images are obtained at different points in the cardiac cycle or over different exposure times. The concatenated information still provides useful data for a clinician or further processing system for assessing the blood velocity distribution within the imaged region.

A more advanced technique for producing a spatial power spectrum is to perform frequency binning on an initial spatial power spectrum (or spectrums) to produce the spatial power spectrum to be output by process 210. This technique allows for higher signal-to-noise ratios and also compensates for different sampling resolutions in the spatial frequency domain by standardizing it into a fixed grid.

In this technique, each laser speckle image may be converted into an initial spatial power spectrum in a step 211. This may comprise, for instance, performing the multi-to-single dimension spatial frequency transform. The initial spatial power spectrum identifies, for each of a plurality of spatial frequencies, a power of the spatial frequency in the (corresponding) laser speckle image.

A plurality of different spatial frequency ranges are then defined in a step 212.

In step 213, for each spatial frequency range, the powers of the spatial frequencies in the initial power spectrum(s) that fall within that spatial frequency range are combined (e.g., summed or averaged) to produce a power for the spatial power spectrum to be output by process 210. Thus, there are as many powers or values in the spatial power spectrum (output by process 210) as there are spatial frequency ranges.

There are various mechanisms for performing step 213 if there is more than one laser speckle image, and therefore more than one initial spatial power spectrum.

In one approach, step 213 comprises combining (e.g., summing or averaging) all initial spatial power spectrums to produce a combined initial spatial power spectrum. Step 213 then, for each spatial frequency range, produces a power for the spatial power spectrum by combining (e.g., summing or averaging) all powers of spatial frequencies, from the combined initial spatial power spectrum, that fall within said spatial frequency range. This produces the spatial power spectrum.

In another approach, step 213 comprises, for each spatial frequency range, producing a set of intermediate powers. Each intermediate power in a same set is a combination (e.g., sum or average) of all powers of spatial frequencies, of a different initial spatial power spectrum, that fall within the corresponding spatial frequency range. The number of intermediate powers in each set is therefore the same as the number of initial spatial power spectrums. Each set of intermediate powers is then processed to produce, for each set, a single power for the spatial power spectrum. In particular, the intermediate powers in a same set are combined (e.g., summed or averaged) to produce a single power for that set.

It is possible that each spatial frequency range may partially overlap one or more other spatial frequency ranges. This provides a windowing effect for improved signal-to-noise ratio.

Alternatively, if there is more than one laser speckle image, it is possible to perform an iteration of step 213 on each initial spatial power spectrum, i.e., perform frequency binning on each initial spatial power spectrum to produce a plurality of intermediate spatial power spectrums. The intermediate spatial power spectrums can then be concatenated together to produce the spatial power spectrum.

It is not essential for only a single spatial power spectrum to be output by process 210 or step 213. Rather, a plurality of spatial power spectrums (e.g., one for each laser speckle image) may be output.

The method 200 also comprises a step 220 of defining, using the spatial frequency data, a plurality of blood velocity indicators, each blood velocity indicator indicating or representing an amount of blood moving at a respective, different blood velocity within the region of interest. Preferably, step 220 comprises defining no fewer than 5 blood velocity indicators, e.g., no fewer than 10 blood velocity indicators.

The relationship between each blood velocity indicator and the amount of blood may not be a direct correlation. Rather, there may be a non-linear relationship between the blood velocity indicator and the amount of blood at a particular blood velocity.

In a simple example, each power in the spatial power spectrum is able to act as a respective blood velocity indicator. This provides numeric representation of blood velocity. In this way, the spatial power spectrum itself comprises or defines the blood velocity indicators produced in step 220.

In another example, each power in the spatial power spectrum may be categorized or classified to produced categorical blood velocity indicators. For instance, each power may be compared to one or more predetermined, non-overlapping ranges. Each range may define a different category or classification (e.g., "Low", "Medium" or "High"). A categorical blood velocity indicator can be generated by identifying into which predetermined range the power falls, and producing a blood velocity indicator responsive to this value.

In a more advanced example, step 210 comprises processing a plurality of laser speckle images to produce, for each laser speckle image, a spatial power spectrum. This may comprise, as previously explained, performing frequency binning (which produces a single value for a spatial frequency range within a spatial power spectrum) using the approach described with reference to Figure 2.

It will be appreciated that each laser speckle image is captured at a different point in time or over a different period of time. This produces a series or sequence of spatial power spectrums that show how the spatial power spectrum changes over time. Thus, it is possible to produce a time-varying signal at each of plurality of spatial frequencies or spatial frequency ranges. Each time-varying signal represents a change of amplitude at a particular spatial frequency or spatial frequency range over time. Thus, each time-varying signal is effectively a spatial-frequency filtered version of the plurality of spatial power spectrums. Each time-varying signal may then undergo a Fourier-based transform to produce a frequency power spectrum for each of the plurality of spatial frequencies or spatial frequency ranges. The amplitude or power at different frequencies of each frequency power spectrum can form or provide a respective blood velocity indicator.

In this way, a plurality of sets of blood velocity indicators can be generated. Each set of blood velocity indicators contains a plurality of blood velocity indicators, which each indicate an amplitude of a different frequency or frequency range for a same spatial frequency or spatial frequency range (associated with that set). Different sets of blood velocity indicators are associated with different spatial frequencies or spatial frequency ranges.

This is conceptually illustrated by Table 1, which demonstrates how different frequency components at different spatial frequencies can be produced. The rows of Table 1 represent different sets of blood velocity indicators, as previously described. The columns of Table 1 represent the blood velocity indicators generated from a respective frequency power spectrum.

TABLE 1

| | Frequency | | |
|---|---|---|---|
| Spatial Frequency | $f_1$ | $f_2$ | $f_3$ |
| $SF_1$ | $SF_1(f_1)$ | $SF_1(f_2)$ | $SF_1(f_3)$ |
| $SF_2$ | $SF_2(f_1)$ | $SF_2(f_2)$ | $SF_2(f_3)$ |
| $SF_3$ | $SF_3(f_1)$ | $SF_3(f_2)$ | $SF_3(f_3)$ |

Thus, if step 210 comprises processing a plurality of laser speckle images to produce, for each laser speckle image, a spatial power spectrum, then step 220 may comprise the following steps: processing the spatial power spectrum to define, for each of a plurality of spatial frequencies or spatial frequency ranges, a time varying value; performing a Fourier-based transform on each time varying value to produce a plurality of frequency power spectrums; for each frequency power spectrum, defining an amplitude of each of a plurality of frequency or frequency ranges of the frequency power spectrum as a blood velocity indicator.

Examples of Fourier-based transforms are well known in the art, and include fast Fourier transforms or short-time fast Fourier transforms.

This advanced approach incorporates time-varying data into each indicator produced. Thus, each indicator can further carry important and clinically interpretable information about the blood velocity in the imaged region. This provides valuable data for increasing the contextual understanding of a blood velocity indicator, e.g., for further processing by an analyzing system or for presentation for an individual's understanding.

Although spatial power spectrums are derived from laser speckle images that are captured over a period of time (i.e., non-instantaneous time steps), the relevant time information contained in a series/sequence of spatial power spectrums is in time steps of a much larger magnitude (e.g., an order of 100x or more) greater than a capture time.

Method 200 may comprise a step 230 of displaying, e.g., at a user interface, the blood velocity indicators defined in step 220. Approaches for controlling a user interface to display data, such as the blood velocity indicators, are well known in the art.

Step 230 may, or instance, comprise displaying (e.g., at a user interface) the spatial power spectrum containing the blood velocity indicators. This approach recognizes that it is possible to visualize the spatial power spectrum as a bar or line diagram. This allows for comparison of the blood velocity distribution between different perfusion states (e.g., within a same patient) or across patients. If this step is performed, this effectively provides a visual representation of the blood velocity indicators.

It is appreciated that, at least to ensure consistent interpretability to information produced using a spatial frequency transform, the resolution of the laser speckle image(s) in terms of surface distance (e.g., measured in mm or m) should be known. This may be particularly relevant for camera-based systems, where the optical sensor can be located at different distances from the surface.

The pixel spacing (e.g., in mm) is preferably used to define the sampling frequency of the spatial frequency transform.

It is possible to estimate the surface distance resolution of a laser speckle image using a variety of techniques, including triangulation or identification of reference markers in the image. Alternatively, the surface distance resolution can be predefined or set, e.g. by ensuring that laser speckle images are captured from a same distance from the/each subject, e.g., using spacers. This approach provide ways of calibrating the frequency content across measurements.

To improve the accuracy of the blood velocity information, the one or more laser speckle images preferably comprises a plurality of laser speckle images having different exposure times. Images recorded with different exposure times will contain blood velocity information with different dynamic ranges.

In general, the powers of the spatial power spectrum produced by process 210 thereby produce a multi-variate marker for the blood velocity distribution and perfusion of the tissue. In particular, the powers (acting as blood velocity indicators) effectively produce an ordered set of magnitudes, the order being defined by the spatial frequency associated with each power.

As previously explained, it is possible to perform one or more pre-processing steps and/or one or more post-processing steps. In particular, for improved interpretability, it is possible to further process the blood velocity indicators (e.g., the spatial power spectrum) to produce other measures or outputs.

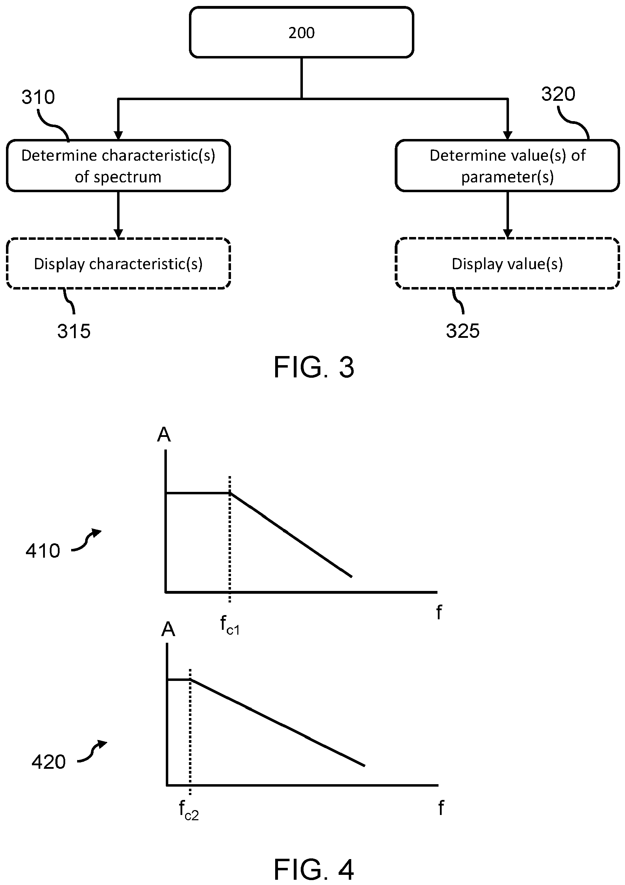

Figure 3 illustrates some example post-processing steps that can be used to process the blood velocity indicators to produce different types of processed data. Thus, Figure 3 illustrates a method 300 that includes various approaches for converting the blood velocity indicators into some target quantity of variables.

Two approaches are illustrated, one represented by step 310 and the other represented by step 320. One or both of these steps may be included in method 300.

One approach is illustrated by step 310, which comprises processing the spatial power spectrum to determine one or more characteristics of the spatial power spectrum. The spatial power spectrum comprises the blood velocity indicators, e.g., each value of the spatial power spectrum is a blood velocity indicator.

One suitable example of the characteristics of the spatial power spectrum include a cut-off or corner spatial frequency of the spatial power spectrum. Another suitable characteristic is a gradient or slope of the decaying attenuation or decay from the cut-off spatial frequency to higher frequencies. The cut-off spatial frequency in the spectrum and the gradient/slope of the decay are indicators for the amount and speed distribution of scatterers in the imaged region.

Figure 4 intuitively illustrates the relationship between speed of scatterers and the spatial power spectrum.

Figure 4 illustrates two waveforms, a first waveform 410 providing a baseline state and a second waveform 420 providing a state in which scatterers (e.g., red blood cells) in the illuminated region of interest have an increased flow compared to the first waveform. Each waveform depicts an amplitude or power A (y-axis) against spatial frequency f (x-axis).

Each waveform thereby provides a visual representation of the spatial frequency data or spatial power spectrum.

When illuminating the region of interest with light, there is a certain likelihood for each illuminating photon to hit one or more scatterers moving in the illuminated region of interest at one or more of different velocities. This likelihood, and therefore the number of photons that will only encounter slower scatterers and/or the number that will encounter fast scatterers, will depend on the available velocity distribution. The velocity distribution will therefore define both the blurring of the laser speckle image, i.e., the attenuation of higher spatial frequency components, as well as the frequency-dependency of the attenuation. This is observable and quantifiable using the cut-off or corner spatial frequency and extent of decay.

In particular, as the number of faster scatterers increases the likelihood of a photon hitting a fast scatterer increases. Thus, the likelihood of smaller spatial frequencies being attenuated increases. This effectively shifts the cut-off spatial frequency of the spatial power spectrum to a lower spatial frequency. This effect is illustrated by how a first cut-off spatial frequency $f_{c1}$ of the first waveform 410 is greater than a second cut-off spatial frequency $f_{c2}$ of the second waveform 420 - as the flow of scatterers (e.g., red blood cells) in the illuminated region increases.

However, although this shift of the cut-off spatial frequency towards a lower spatial frequency results from the adding of a few fast scatterers, it could also result from an increase in the number of moderately fast scatterers. The differences between these scenarios could be disentangled by an approach that looks at the slope of the decay, for instance, which would be different in the different scenarios.

The faster the scatterers, the quicker high spatial frequencies will decorrelate. This implies that the slope (e.g., of the waveform) will be steeper with an increasing ratio of fast scatterers to slow scatters, as the attenuation of higher powers grows with faster scatterers. The relationship between this ratio and the steepness or gradient of the waveform may be a non-linear relationship.

Figure 4 thereby demonstrates one advantage of determining characteristics of the spatial power spectrum, as well as an advantage in the spatial power spectrum itself (e.g., for viewing by an individual for assessment).

Turning back to Figure 3, the method 300 may further comprise a step 315 of displaying the determined characteristic(s) of the spatial power spectrum at a user interface. Approaches for controlling a user interface to display data are well known in the art.

Step 320 illustrates another approach for processing the blood velocity indicators.

Step 320 comprises processing one or more of the blood velocity indicators using a computer-implemented function or a mapping function to determine the value(s) of one or more parameter(s) of the subject.

Example parameters that could be derived, predicted or determined using a computer-implemented method include a blood velocity distribution or distributional parameters thereof. The computer-implemented function may thereby be a function that maps values of blood velocity indicators into a blood velocity distribution.

Other example parameters that could be derived, predicted or determined using a computer-implemented method include one or more clinical parameters, such as a pathology prognosis (e.g., a predicted change for reducing ischemia or for a wound to heal).

The computer-implemented function may, for instance, be a machine-learning algorithm. A first example of a machine-learning algorithm is configured to process blood velocity indicators to produce or determine a blood velocity distribution or distribution parameters thereof. A second example of a machine-learning algorithm is configured to process blood velocity indicators to produce or determine one or more values for one or more clinical parameters, such as a pathology prognosis.

A machine-learning algorithm is any self-training algorithm that processes input data in order to produce or predict output data. For the first example of the machine-learning algorithm, the input data comprises blood velocity indicators and the output data comprises blood velocity distribution and/or distributional parameters thereof. For the second example of the machine-learning algorithm, the output data instead comprises one or more values for one or more clinical parameters.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian models are suitable alternatives. The precise decision as to which machine-learning algorithm is selected may be at least partially dependent upon the data type to be processed or produced (e.g., whether the output data is a continuous or categorical variable). Appropriate selection will be apparent to the skilled person in the field of machine-learning algorithms.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can be repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

For generating the first example of the machine-learning algorithm, the training input data entries of the training dataset correspond to example sets of blood velocity indicators and the training output data entries of the training dataset correspond to blood velocity distributions or distribution parameters thereof.

One approach for generating such a training dataset is to make use of a Monte-Carlo simulation. In particular, different simulated laser speckle images can be produced (using a Monte-Carlo technique) using a tissue model and ground-truth blood velocity distribution and/or distributional parameters thereof. The simulated laser speckle images can then be processed (using above-described techniques) to produce a set of blood velocity indicators for each simulated laser speckle image. Each set of blood velocity indicators acts as a training input data entry. Each ground-truth blood velocity distribution and/or distributional parameters thereof used to simulate the simulated laser speckle images acts a training output data entry.

This technique provides a simple and easily implementable end-to-end learnable model. In particular, despite lacking an analytical model for the underlying functional relationship, this approach can be used to approximate the outcome of such an unknown functional relationship.

For generating the second example of the machine-learning algorithm, the training dataset is a first training dataset that comprises, as training input data entries, sets of blood velocity indicators and, as training output data entries, corresponding values for one or more clinical parameters (for the patient for whom the blood velocity indicators of the corresponding training input data entry was generated). The values for the one or more clinical parameters may be produced by a skilled and/or experienced clinician and/or based on historical medical data.

In some examples, the second example of the machine-learning algorithm comprises using two machine-learning algorithmic processes. Each machine-learning algorithmic process is another example of a machine-learning algorithm. A first machine-learning algorithmic process may be identical to the first example of the machine-learning algorithm previously described, and may be trained as previously described. The second machine-learning algorithmic process may process the output of the first example of the machine-learning algorithm, using another machine-learning algorithm, to predict values for one or more clinical parameters.

In this approach, the training dataset may again comprise, as training input data entries, sets of blood velocity indicators and, as training output data entries, corresponding values for one or more clinical parameters. For this scenario, training the second example of the machine-learning algorithm may comprise modifying only the second machine-learning algorithmic process, and not the already trained first machine-learning algorithmic process. This increases an ease of training the second machine-learning algorithmic process.

The method 300 may further comprise a step 325 of displaying the determined value(s) of the parameter(s) at a user interface. Approaches for controlling a user interface to display data are well known in the art.

It will be appreciated that step 320 may be repeated for different parameters. Thus, more than one machine-learning algorithm may process (at least) the blood velocity information to produce the value(s) of the parameter(s) for the subject under investigation.

Above-described approaches make use of only the blood velocity indicators derived from laser speckle images to produce processed data (e.g., blood velocity distribution information and/or the value(s) for one or more clinical parameters).

However, it is possible to use further information to produce the processed data.

One suitable example of such further information is subject characteristics, e.g., characteristics of the subject such as age, comorbidities, subcutaneous fat content, number of chromophores and/or different macroscopic morphology characteristics such as the presence and/or absence of scar/wound tissue.

It is recognized that the scattering properties of the illuminated region of interest of the subject will impact or bias the baseline appearance of the projected speckle pattern (e.g., regardless of the actual blood velocities). The scattering properties are dependent or upon characteristics of the subject, such as those previously described. Thus, the blood velocity indicators produced by proposed methods have a relationship with subject characteristics subject. Using such subject characteristics when determining one or more parameters of the subject allow these to be taken into account, e.g., as control variables.

Accordingly, the value(s) of one or more parameters of the subject (e.g., blood velocity information and/or clinical parameters) may be determined further using a set of one or more subject characteristics. This provides a more accurate and reliable approach for determining such value(s).

In a simple example, the set of one or more subject characteristics may be included in input data provided to a computer-implemented method used for generating the one or more parameters of the subject. For instance, a machine-learning method used for determining the value(s) of the one or more parameters may be trained to receive, as input, the blood velocity parameters and the set of one or more subject characteristics. In this case, each training input data entry of the training dataset used to train the machine-learning algorithm may further include the set of one or more subject characteristics of the subject associated with the training input data entry.

In a more complex example, a different machine-learning method may be trained for each of a plurality of clusters of historic subjects, e.g., each machine-learning algorithm may be trained using data derived from the corresponding single cluster of historic subjects. Each cluster of historic subject may share similar values for the one or more subject characteristics. The one or more subject characteristics of the current subject may be processed to identify a most similar cluster of historic subjects. The machine-learning algorithm trained for/using that cluster of historic subjects may then be used to process (at least) the blood velocity indicators of the current subject to produce the value(s) for one or more parameter(s) of the subject.

Another suitable example of further information is information derived from a time-varying signal representing a temporal variation of blood flow in the region of interest. A suitable example of a time-varying signal is a photoplethysmography signal or photoplethysmogram, e.g., obtainable from a PPG sensor such as a finger-worn pulse oximeter. Another example of a time-varying signal is a speckle plethysmography signal.

While the spatial frequency data can be used to evaluate the average velocity distribution, the time-varying signal can capture pulsatile aspects along time dimension. This provides additional, clinically useful information for assessing and determining output data.

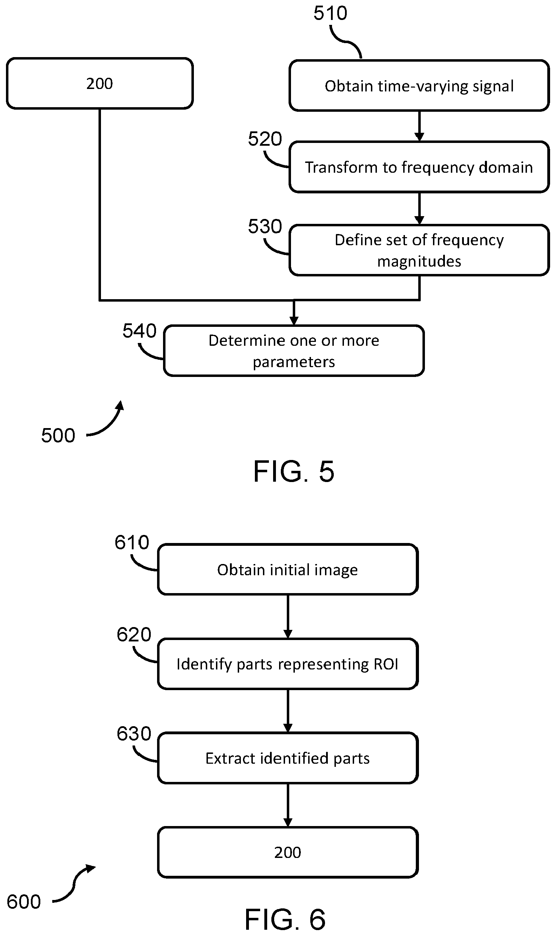

Figure 5 illustrates a method 500 according to an embodiment, which makes use of a time-varying signal in producing output data.

The method 500 comprises performing the method 200 previously described, e.g., with reference to Figure 2.

The method 500 also comprises a step 510 of obtaining a time-varying signal. The time-varying signal represents a temporal variation of blood flow in the region of interest. Suitable examples are known to the skilled person, some of which have been previously described.

The method 500 also comprises a step 520 of transforming the time-varying signal to the frequency domain. Step 520 can be performed using any established transformation technique, e.g., any suitable Fourier-based transform such as a fast Fourier transform or a short-time fast Fourier transform.

The method 500 also comprises a step 530 of defining a set of frequency magnitudes. The set of frequency magnitudes contains magnitudes of different frequency components or frequency ranges of the time-varying signal. In one example, the set of frequency magnitude comprises the values/magnitude of each of a plurality of predetermined frequencies. In another example, the set of frequency magnitudes comprises, for each of a plurality of predetermined frequency ranges, the summed or average (i.e., the combined) values/magnitude of the magnitudes within said predetermined frequency range.

The method 500 further comprises a step 540 of using the blood velocity indicators and the defined set of frequency magnitudes to determine one or more parameters of the illuminated region of interest.

The one or more parameters may comprise, for instance, blood velocity distribution information and/or clinical parameters (such as a prognosis or the like).

Approaches for processing data to produce such one or more parameters have been previously described in the context of processing blood velocity indicators alone, e.g., using a machine-learning algorithm. These approaches may be appropriately adapted for the processing of additional information as input, e.g., the defined set of frequency magnitudes as well as the blood velocity indicators.

In this case, each training input data entry of the training dataset used to train the machine-learning algorithm may further include a set of frequency magnitudes derived from a time-varying signal.

Preferably, the time-varying signal obtained in step 510 was captured over at least the exposure time used for generating the laser speckle image.

To improve the signal-to-noise ratio of the spatial power spectrum and/or the blood velocity information, as well as the accuracy of any output data produced using such information, the content of the laser speckle image(s) can be controlled or defined.

Two general approaches for defining the content of the laser speckle image(s) are considered. Of course, both, one or none of these approaches may be performed depending upon the embodiment.

A first approach processes, masks or filters the obtained laser speckle image(s) to produce one or more processed laser speckle images that are used in the generation of the spatial frequency data. Thus, in the first approach, content is extracted from obtained laser speckle image(s). In other words, one or more pre-processing steps are applied in order to generate the laser speckle image(s) that are processed using the spatial frequency transform.

A second approach controls how and/or when the laser speckle image(s) are captured. Thus, in the second approach, the operation of the laser speckle device is controlled. The second approach may, for instance, control an exposure time of the laser speckle image(s) and/or a time at which the laser speckle image(s) is/are captured.

Figure 6 illustrates a method 600 for carrying out an approach according to the first approach for defining the content of the laser speckle image(s).

The method 600 comprises a step 610 of obtaining an initial laser speckle image. The initial laser speckle image contains a speckle pattern produced by the reflection of light illuminating a region of interest of a subject. The initial laser speckle image contains the laser speckle image and has a higher resolution than the laser speckle image.

The method 600 also comprises a step 620 of processing the initial laser speckle image to identify one or more parts of the initial laser speckle image that represent the region of interest.

Step 620 may be performed, for instance, by identifying, as heterogeneous parts, any parts of the initial laser speckle image that represent heterogeneous regions of the subject that contain one or more blood capillaries and one or more of: an artery and a vein; and excluding the identified heterogeneous parts of the initial laser speckle image from the identified one or more parts of the initial laser speckle image that represent the region of interest.

Detection of heterogeneous regions can be performed by analyzing, for instance, textural features across locations in the initial speckle image. A clustering or segmentation into a subset of homogenous regions can then allow for sub-region selection, e.g. the discarding or exclusion of areas with an underlying larger vein in favor of sub-regions dominated by dermal tissue.

The method 600 also comprises a step 630 of extracting the identified one or more parts of the initial laser speckle image to define the laser speckle image.

The method 600 provides a mechanism by which an area of interest is manually or automatically masked.

More particularly, the initial laser speckle image (e.g., recorded by an optical sensor) will cover a certain area. Within this area a region of interest can be selected. This selection can be performed manually (e.g., using spline interpolated control points) or (preferably) automatically.

The masking can have functional relevance in the event of case of heterogeneous surfaces where e.g. a larger vessel passes through the observed area. This could lead to misinterpretation or inaccuracies as a spectral analysis of a small patch will always assume a periodic continuation of the observed pattern. It would therefore be advantageous to detect and exclude/mask severe heterogeneity from the laser speckle image(s).

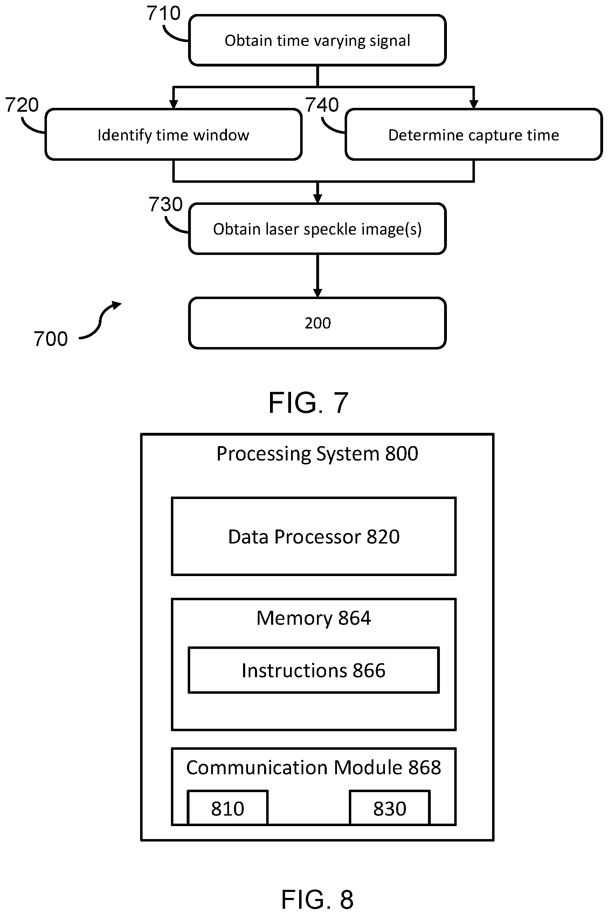

Figure 7 illustrates a method 700 for carrying out an approach according to the second approach for defining the content of the laser speckle image(s). In particular, Figure 7 illustrates a technique that controls the time at which the laser speckle image(s) are captured and/or the capture time of the laser speckle image(s).

The method 700 comprises a step 710 of obtaining a time varying signal. The time-varying signal represents a temporal variation of blood flow in the region of interest. Suitable examples are known to the skilled person, some of which have been previously described.

Step 710 is analogous to step 510 previously described. Step 710 may be performed by obtaining the time varying signal from a monitoring device such as a PPG sensor, e.g., such as a finger-worn pulse oximeter.

The method 700 further comprises a step 720 of identifying a time window for capturing the laser speckle image(s). In particular, the time varying signal may be processed to identify a pulsatility (index) or periodicity of the time varying signal. The periodicity of the time varying signal can be used to set the time window during which the laser speckle image is to be captured.

The identified time window may, for instance, be a time window corresponding to the periodicity of the time varying signal (e.g., a periodicity due to motion or heartbeat(s)), e.g., a time window having a length proportional to a period of the time varying signal.

As another example, the identified time window may be a time window that includes a known or predetermined number of periods, or predetermined proportion of a period, of the time varying signal.

As yet another example, the identified time window may be a time window that includes the same number of periods, of proportion of period, of a previous time window during which a previous or earlier laser speckle image was captured. This ensures consistency in measuring or obtaining the laser speckle image(s).

The method further comprises a step 730 of obtaining or capturing one or more laser speckle image(s) in the identified time window. Step 730 may be performed through appropriate control of the laser speckle device.

The time varying signal obtained in step 710 may be further used in determining one or more parameters, e.g., as previously described with reference to Figure 5.

Method 700 may comprise a step 740 processing the time varying signal to determine a capture time for the laser speckle image(s). In particular, the time varying signal may be processed using a frequency transform process (e.g., a Fourier-based transform) to identify one or more frequency components of the time varying signal (e.g., one or more dominant frequencies).

Step 730 may be modified to comprise obtaining the laser speckle image(s) with an exposure time that matches one of the frequency components or dominant frequencies, e.g., the inverse of one of the frequency components or dominant frequencies.

This approach can, for instance, ensure that a plurality of laser speckle images are captured at a same point during a periodic cycle, such as a cardiac cycle, of the subject.

In some examples, step 720 can be omitted. Step 730 can be modified accordingly.

Above described approaches and methods produce output data (e.g., the blood velocity indicator or data derived therefrom) from a single set of one or more laser speckle images. To provide improved clinical insight, two sets of output data may be generated, with a first set of output data being generated before a clinical interrogation or stimulus and a second set being generated after the clinical interrogation or stimulus. Examples stimuli include a thermal or occlusive stimulus.

In this way, any previously described method may be repeated either side of a clinical stimulus. The output data produced by the repeated method is preferably provided or displayed at a user interface. This provides useful insights into modulation capacity of the tissue.

Figure 8 is a schematic diagram of a processing system according to an embodiment, e.g., the processing system 150 previously described with reference to Figure 1.

As shown, the processing system 800 may include data processor 820, a memory 864, and a communication module 868. These elements may be in direct or indirect communication with each other, for example via one or more buses.

The data processor 820 may include a central data processor (CPU), a digital signal processor (DSP), an ASIC, a controller, an FPGA, another hardware device, a firmware device, or any combination thereof configured to perform the operations described herein. The data processor 820 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 864 may include a cache memory (e.g., a cache memory of the data processor 820), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and non-volatile memory, or a combination of different types of memory. In an embodiment, the memory 864 includes a non-transitory computer-readable medium. The non-transitory computer-readable medium may store instructions. For example, the memory 864, or non-transitory computer-readable medium may have program code recorded thereon, the program code including instructions for causing the processing system 800, or one or more components of the processing system 800, particularly the data processor 820, to perform the operations described herein. For example, the processing system 800 can execute operations of the method 200.

Instructions 866 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 864, with the code recorded thereon, may be referred to as a computer program product.

The communication module 868 can include any electronic circuitry and/or logic circuitry to facilitate direct or indirect communication of data between the processing system 800 and any external devices.

In that regard, the communication module 868 comprises an input interface 810 and an output interface 830 for the processing system 800. The input interface 810 may be configured to receive input data for the data processor, e.g., the one or more laser speckle images and optionally the time varying signal. The output interface 830 may be configured to provide output data from the data processor, e.g., the determined blood velocity indicators, parameters produced by processing the blood velocity indicators and so on.

In some instances, the communication module 868 facilitates direct or indirect communication between various elements of the processing system 800, e.g., using a bus or the like.

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored there on the computer program (product) previously described. There is also proposed a data carrier signal carrying the computer program (product) previously described.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

**Claims**

1. A computer-implemented method for processing one or more laser speckle images, each laser speckle image being an image containing a speckle pattern produced by the reflection of light illuminating a region of interest of a subject, the computer-implemented method comprising:

   processing the one or more laser speckle images using a spatial frequency transform, to thereby produce spatial frequency data; and
   defining, using the spatial frequency data, a plurality of blood velocity indicators, each blood velocity indicator indicating or representing an amount of blood moving at a respective, different blood velocity within the region of interest.

2. The computer-implemented method of claim 1, wherein:

   the step of processing the one or more laser speckle images to produce spatial frequency data comprises converting the one or more laser speckle images into a spatial power spectrum identifying, for each of a plurality of spatial frequencies, a power of the spatial frequency in the one or more laser speckle images; and
   each power in the spatial power spectrum acts as a respective blood velocity indicator.

3. The computer-implemented method of claim 2, wherein the step of processing the laser speckle image to produce spatial frequency data comprises:

   converting each laser speckle image into an initial spatial power spectrum identifying, for each of a plurality of spatial frequencies, a power of the spatial frequency in the laser speckle image;
   defining a plurality of spatial frequency ranges; and

producing the spatial power spectrum by, for each of the plurality of spatial frequency ranges, combining the powers of the spatial frequencies, of each initial spatial power spectrum, that fall in the spatial frequency range to produce a power for the spatial power spectrum.

4. The computer-implemented method of any of claims 1 to 3, further comprising processing one or more of the blood velocity indicators using a computer-implemented method to determine one or more values for one or more characteristics of the subject, wherein the one or more characteristics comprise at least one of: a blood velocity distribution; a clinical parameter such as a pathology prognosis.

5. The computer-implemented method of claim 4, wherein the one or more characteristics comprises at least one pathology prognosis.

6. The computer-implemented method of claim 4 or 5, wherein the computer-implemented model is a machine-learning model.

7. The computer-implemented method of any of claims 1 to 6, further comprising:

   obtaining a time-varying signal representing a temporal variation of blood flow in the region of interest;
   transforming the time-varying signal to the frequency domain;
   defining a set of frequency magnitudes which contains magnitudes of different frequency components or frequency ranges of the time-varying signal; and
   using the blood velocity indicators and the defined set of frequency magnitudes to determine one or more parameters of the illuminated region of interest.

8. The computer-implemented method of any of claims 1 to 7, further comprising a laser speckle image defining process comprising, for each laser speckle image:

   obtaining an initial laser speckle image, being an image containing a speckle pattern produced by the reflection of light illuminating a region of interest of a subject, wherein the initial laser speckle image contains the laser speckle image and has a higher resolution than the laser speckle image;
   processing the initial laser speckle image to identify one or more parts of the initial laser speckle image that represent the region of interest; and
   extracting the identified one or more parts of the initial laser speckle image to define the laser speckle image.

9. The computer-implemented method of claim 8, wherein the step of processing the initial laser speckle image comprises:

   identifying, as heterogeneous parts, any parts of the initial laser speckle image that represent heterogeneous regions of the subject that contain one or more blood capillaries and one or more of: an artery and a vein; and
   excluding the identified heterogeneous parts of the initial laser speckle image from the identified one or more parts of the initial laser speckle image that represent the region of interest.

10. The computer-implemented method of claim 8 or 9, wherein the step of processing the initial laser speckle image comprises identifying one or more parts of the initial laser speckle image that contain one or more desired textual features of the initial laser speckle image as the parts of the initial laser speckle image that represent the region of interest.

11. The computer-implemented method of any of claims 1 to 10, wherein the one or more laser speckle images comprises a plurality of laser speckle images having different exposure times.

12. A computer program product comprising code which, when executed by a processor circuit, causes the processor circuit to perform the steps of the method according to any of claims 1 to 11.

13. A non-transitory computer-readable medium or data carrier comprising or carrying the computer program product of claim 12.

14. A processing system processing one or more laser speckle images, each laser speckle image being an image containing a speckle pattern produced by the reflection of light illuminating a region of interest of a subject, the processing system being configured to:

process the one or more laser speckle images using a spatial frequency transform, to thereby produce spatial frequency data; and

define, using the spatial frequency data, a plurality of blood velocity indicators, each blood velocity indicator indicating or representing an amount of blood moving at a respective, different blood velocity within the region of interest.

15. A laser speckle imaging system comprising:

the processing system of claim 14; and
a laser speckle device configured to capture the one or more laser speckle images.

160

150

115

140

100

110

FIG. 1

210

211

115

212

Produce initial spatial power spectrum

Define frequency ranges

Produce spatial power spectrum

213

200

220

Define blood velocity indicators

Display blood velocity indicators

230

FIG. 2

200

310

Determine characteristic(s)
of spectrum

320

Determine value(s) of
parameter(s)

Display characteristic(s)

315

Display value(s)

325

## FIG. 3

A

410

$f_{c1}$

f

A

420

$f_{c2}$

f

## FIG. 4

510

200

Obtain time-varying signal

520

Transform to frequency domain

530

Define set of frequency magnitudes

540

Determine one or more parameters

500

FIG. 5

610

Obtain initial image

620

Identify parts representing ROI

630

Extract identified parts

200

600

FIG. 6

710 — Obtain time varying signal

720 — Identify time window 740 — Determine capture time

730 — Obtain laser speckle image(s)

200

700

## FIG. 7

Processing System 800

Data Processor 820

Memory 864

Instructions 866

Communication Module 868

810 830

## FIG. 8

Europäisches
Patentamt

European
Patent Office

Office européen
des brevets

# EUROPEAN SEARCH REPORT

**Application Number**

EP 22 19 4618

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 9 970 955 B1 (HOMYK ANDREW [US] ET AL) 15 May 2018 (2018-05-15) * column 5, lines 21-43 * * page 10, lines 42-56 * * column 14, lines 10-17 * * column 21, lines 29-64 * * column 22, lines 15-56 * * column 27, lines 8-27 * * column 29, lines 43-61 * * column 36, lines 17-26 * * figures 1-4, 6 * | 1-4,6-15 | INV. A61B5/026 A61B5/00 |
| X | WO 2009/008745 A2 (IND RES LTD [NZ]; THOMPSON OLIVER BENDIX [NZ] ET AL.) 15 January 2009 (2009-01-15) * paragraphs [0013], [0029], [0031], [0035], [0083], [0084], [0094], [0098], [0099], [0103], [0104], [0139] * * figures 1, 3 * | 1-4,7,8, 11-15 | |
| X | US 2021/177283 A1 (WHITE SEAN MICHAEL [US] ET AL) 17 June 2021 (2021-06-17) * paragraphs [0005] - [0007], [0025], [0078], [0092] * | 1-4,7, 12-15 | TECHNICAL FIELDS SEARCHED (IPC)  A61B |

The present search report has been drawn up for all claims

| Place of search | Date of completion of the search | Examiner |
|---|---|---|
| The Hague | 10 February 2023 | Worms, Georg |

EPO FORM 1503 03.82 (P04C01)

## ANNEX TO THE EUROPEAN SEARCH REPORT
## ON EUROPEAN PATENT APPLICATION NO.

**EP 22 19 4618**

This annex lists the patent family members relating to the patent documents cited in the above-mentioned European search report.
The members are as contained in the European Patent Office EDP file on
The European Patent Office is in no way liable for these particulars which are merely given for the purpose of information.

**10-02-2023**

| Patent document cited in search report | | Publication date | Patent family member(s) | | Publication date |
|---|---|---|---|---|---|
| US 9970955 | B1 | 15-05-2018 | US | 9970955 B1 | 15-05-2018 |
| | | | US | 10871503 B1 | 22-12-2020 |
| WO 2009008745 | A2 | 15-01-2009 | CN | 101784227 A | 21-07-2010 |
| | | | EP | 2166934 A2 | 31-03-2010 |
| | | | JP | 2010532699 A | 14-10-2010 |
| | | | US | 2011013002 A1 | 20-01-2011 |
| | | | WO | 2009008745 A2 | 15-01-2009 |
| US 2021177283 | A1 | 17-06-2021 | EP | 3838129 A1 | 23-06-2021 |
| | | | US | 2021177283 A1 | 17-06-2021 |

EPO FORM P0459

For more details about this annex : see Official Journal of the European Patent Office, No. 12/82